# EUROPÄISCHE PATENTSCHRIFT

(11) **EP 2 061 407 B1**
(45) Veröffentlichungstag und Bekanntmachung des Hinweises auf die Patenterteilung: **15.06.2011**
(21) Anmeldenummer: 07802138.3
(22) Anmeldetag: 05.09.2007
(51) Int. Cl.: A61F 5/01

(54) **SPRUNGGELENKORTHESE**
ANKLE JOINT ORTHESIS
ORTHÈSE D'ARTICULATION DE CHEVILLE

(30) Priorität: 05.09.2006 DE 102006041195
(43) Veröffentlichungstag der Anmeldung: 27.05.2009
(73) Patentinhaber: Bauerfeind AG, 07937 Zeulenroda (DE)
(72) Erfinder: HESS, Heinrich, 66271 Kleinblittersdorf (DE); SCHEUERMANN, Rainer, 24223 Rainsdorf (DE); KRAUSE, Wolfgang, 36145 Hofbieber (DE)
(74) Vertreter: Bardehle, Heinz
(86) Internationale Anmeldenummer: PCT/EP2007/007731
(87) Internationale Veröffentlichungsnummer: WO 2008/028643

(56) Entgegenhaltungen:
- WO-A-98/29060
- DE-A1- 4 112 069
- DE-U1- 8 617 783
- US-A1- 2004 034 316

## Beschreibung

Die Erfindung bezieht sich auf eine Sprunggelenkorthese, die einen aus biegsamem Material bestehenden, U-förmigen Stützbügel enthält, dessen Schenkel unterhalb des Fußes in einem sich als Lasche in Richtung Mittelfuß erstreckenden Steg zusammenlaufen, über die Knöchel hinausreichen und in ihrem Endbereich durch ein das Bein umschlingendes Befestigungsband zusammengehalten sind. Eine derartige Orthese ist in der DE 41 12 069 A1 beschrieben. Die Gestaltung dieser Sprunggelenkorthese dient dazu, durch Verstärkung ihrer Stützwirkung einen fortschreitenden Heilungsprozess zu verbessern.

Der Erfindung liegt die Aufgabe zugrunde, die Stützwirkung der Orthese weiterhin zu verbessern. Erfindungsgemäß geschieht dies dadurch, dass die Lasche eine ballige Wölbung aufweist, die aufgrund einer Vorspannung der Lasche auf das Würfelbein des Fußes drückt.

Diese Gestaltung der erfindungsgemäßen Sprunggelenkorthese macht sich einen Effekt zunutze, der darin besteht, dass im Bereich der Fußsohle Bereiche existieren, die aufgrund von Druck und entsprechender Weiterleitung eines entsprechenden Signals über die Nerven der betreffenden Person Reflexbewegungen des Fußes auslösen, durch die der Fuß durch Muskeleinsatz weitgehend in seine Normallage gebracht wird. Diesen Effekt nutzt die Erfindung dadurch aus, dass sie bei Druck auf das Würfelbein in den betreffenden Nerven des Fußes ein Signal auslöst, das ein unwillkürliches Anziehen des Fußes bewirkt, womit dieser gegebenenfalls wieder in seine Normallage zurückbewegt wird. Dabei werden im Bereich des Würfelbeines verlaufende Sehnen unter Vorspannung gesetzt und beschleunigen damit entsprechendes Anspringen der zugehörigen Muskeln.

Die Lasche kann man zweckmäßig als von dem Steg in Richtung Vorderfuß weisender, sich entlang der Fußaußenseite der Sohle erstreckender Vorsprung ausbilden, der vor dem Mittelfuß endet und gegenüber dem Steg abbiegbar ist. Diese Gestaltung ist für den Träger einer derartigen Sprunggelenkorthese besonders angenehm, da die Lasche nur einen geringen Raum unterhalb der Wölbung des Fußes einnimmt und somit nicht als hinderlich empfunden wird. Zweckmäßig gestaltet man Lasche und Steg in der Weise, dass die beiden Teile aus unterschiedlichem Material und mit unterschiedlicher Elastizität bestehen.

Eine vorteilhafte Gestaltung von Steg und Lasche lässt sich auch dadurch herbeiführen, dass die Lasche und der Steg einstückig geformt sind, wobei die Lasche gegenüber dem Steg geschwächt ist. Eine derartige Ausführungsform lässt sich in einem Spritzvorgang durchführen, womit also für die Herstellung der Sprunggelenkorthese ein bekanntes und wirksames Kommunikationsmittel gewählt werden kann.

Die Akzeptanz der Sprunggelenkorthese lässt sich dadurch weiterhin verbessern, dass die Schenkel mit dem Steg und die Lasche auf der dem Körper zugewandten Seite von einem Polsterstoff abgedeckt sind. In diesem Fall schmiegt sich gewissermaßen die Sprunggelenkorthese an das Bein und den Fuß des Trägers an, ohne dass dieser sich durch die angelegte Sprunggelenkorthese irgendwie belästigt fühlt.

Um die Sprunggelenkorthese an verschieden breite Füße anpassen zu können, gestaltet man Steg und Schenkel zweckmäßig so, dass sich der Steg so weit in die Schenkel erstreckt, dass seine Länge an die Fußbreite des jeweiligen Trägers durch entsprechende Biegung des Stegs gegenüber den Schenkeln anpassbar ist. Bei Anlegen der Sprunggelenkorthese an einen schmalen Fuß erstrecken sich dann die Biegestellen des Stegs weiter in den Schenkel hinein, während bei Anlegen der Sprunggelenkorthese an einen breiten Fuß die Biegestelle mehr in Richtung unter dem Fuß liegenden Steg wandert.

Um das Tragen der Sprunggelenkorthese weiter angenehm zu gestalten, gestaltet man diese vorteilhaft so, dass an der Innenseite des Außenschenkels ein Polster vorgesehen ist.

In den Figuren ist ein Ausfuhrungsbeispiel der Erfindung dargestellt. Es zeigen:
- Figur 1: die Sprunggelenkorthese angelegt an einem Fuß;
- Figur 2: eine Ansicht des Fußes mit der gemäß Figur 1 angelegten Sprunggelenkorthese von der Außenseite des Fußes gesehen;
- Figur 3: eine der Figur 2 entsprechende Darstellung mit Blickrichtung auf die Innenseite des Fußes.

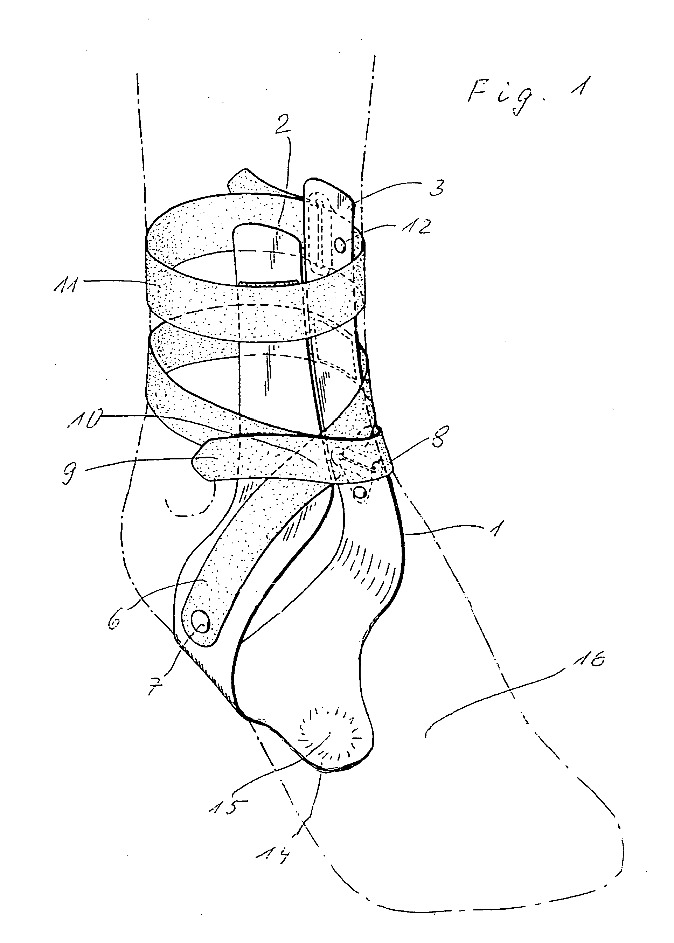

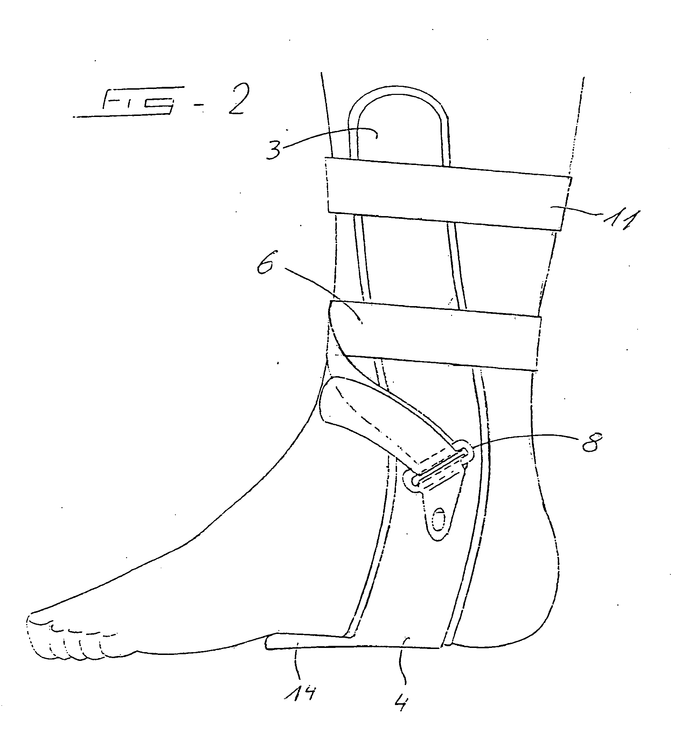

Die in Figur 1 dargestellte Sprunggelenkorthese enthält den U-förmigen Stützbügel 1, der aus den beiden Schenkeln 2 und 3 sowie dem die Schenkel verbindenden Steg 4 besteht. Im unteren Bereich des Schenkels 2 ist das aus einem Klettband bestehende Befestigungsband 6 angebracht, das hier durch den Niet 7 mit dem Schenkel 2 fest verbunden ist. Das Befestigungsband 6 ist oberhalb des Sprunggelenks um das Bein geschlungen und durch eine gestrichelt gezeichnete Umlenköse 8 (siehe Figur 2) zurückgeführt und reicht mit seinem Ende 9 über die Kreuzungsstelle 10 des Befestigungsbandes 6 hinaus, wo es nach Art eines Klettverschlusses an das Befestigungsband 6 angedrückt ist und von diesem gehalten wird. Außerdem weist die Sprunggelenkorthese das Befestigungsband 11 aus, das sich um das Bein oberhalb des Sprunggelenkes schließt und mit seinem einen Ende an dem Schenkel 3 mittels des Nietes 12 befestigt ist. Das andere Ende 13 des Befestigungsbandes 11 ist dann an dieses angedrückt und bildet an der Andruckstelle einen Klettverschluss, womit es nach Umschlingung der oberen Enden der beiden Schenkel 2 und 3 festgehalten ist. Bei dieser Art der Befestigung des U-förmigen Stützbügels 1 handelt es sich um eine bekannte Gestaltung, wie sie aus der oben erwähnten DE 41 12 069 A1 hervorgeht.

Der Steg 4 besitzt die von ihm in Richtung Zehen weisende Lasche 14, die im Bereich des Würfelbeins des Fußes des Trägers der Sprunggelenkbandage die ballige Wölbung 15 aufweist, die eine Vorspannung in Richtung auf das Würfelbein besitzt und auf dieses drückt. Dabei ist die Lage der Lasche 14 so gewählt, dass sie als entlang der Fußaußenseite sich erstreckender Vorsprung angesetzt ist, der vor dem Mittelfuß endet und gegenüber dem Steg 4 abbiegbar ist. Der Mittelfuß ist dabei durch die gestrichelt gezeichnete Linie hinsichtlich seines Umfangs angedeutet, der Bereich des Mittelfußes ist mit dem Bezugszeichen 16 versehen. Durch die vorstehend erläuterte ballige Wölbung 15 wird der in der obigen Einleitung beschriebene Effekt ausgelöst, der eine entsprechende Ausrichtung des Fußes des Trägers der Sprunggelenkbandage verursacht.

In der Figur 2 ist die am Fuß angelegte Sprunggelenkbandage gemäß Figur 1 in Betrachtungsrichtung auf die Fußinnenseite dargestellt. Die Figur 2 zeigt den inneren Schenkel 3 der Fußgelenkbandage und den Steg 4, an den sich die Lasche 14 anschließt. Die Lasche 14 wird in Richtung vom Steg weg ständig dicker und bewirkt damit den gewünschten Druck auf das Würfelbein des Fußes.

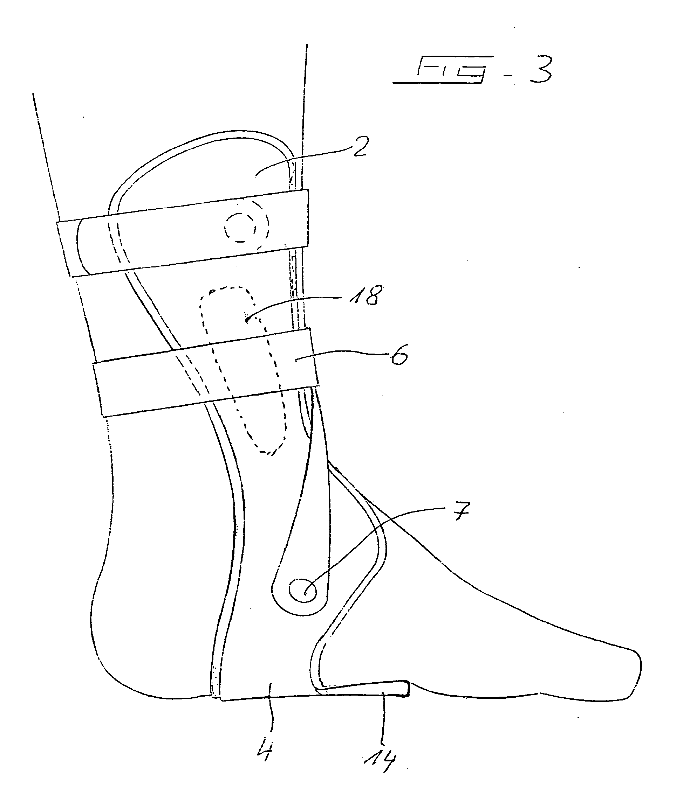

Figur 3 zeigt die Darstellung der am Fuß angelegten Fußgelenkorthese mit Blickrichtung auf die Außenseite des Fußes. In dieser Blickrichtung erscheint die Lasche 14 mit in Richtung zu den Zehen zunehmender Dicke, wodurch der gewünschte Druck auf das Würfelbein erzielt wird. Zu Figur 3 sei noch darauf hingewiesen, dass in dieser Darstellung der Schenkel 3 an seinem oberen Ende etwas verbreitert dargestellt ist, was eine gewisse Abweichung von der Darstellung in Figur 1 bedeutet. Diese Verbreiterung des Endes des Schenkels 2 macht das Tragen der Fußgelenkorthese am Fuß angenehmer. Auf ihrer Innenseite ist das Polster 18 vorgesehen.

## Patentansprüche

1. Sprunggelenkorthese, die einen aus biegsamem Material bestehenden, U-förmigen Stützbügel (1) enthält, dessen Schenkel (2, 3) unterhalb des Fußes in einem sich als Lasche (14) in Richtung Mittelfuß erstreckenden Steg (4) zusammenlaufen, über die Knöchel hinausreichen und in ihrem Endbereich durch ein das Bein umschlingendes Befestigungsband (6, 11) zusammengehalten sind, **dadurch gekennzeichnet, dass** die Lasche (14) eine ballige Wölbung (15) aufweist, die aufgrund einer Vorspannung der Lasche (14) auf das Würfelbein des Fußes drückt.

2. Sprunggelenkorthese nach Anspruch 1, **dadurch gekennzeichnet, dass** die Lasche (14) als von dem Steg (4) in Richtung Vorderfuß weisender, sich entlang der Fußaußenseiten der Sohle erstreckender Vorsprung angesetzt ist, der vor dem Mittelfuß endet und gegenüber dem Steg (4) abbiegbar ist.

3. Sprunggelenkorthese nach Anspruch 1 oder 2, **dadurch gekennzeichnet, dass** die Lasche (14) und der Steg (4) aus unterschiedlichem Material mit unterschiedlicher Elastizität bestehen.

4. Sprunggelenkorthese nach Anspruch 1 oder 2, **dadurch gekennzeichnet, dass** die Lasche (14) und der Steg (4) einstückig geformt sind, wobei die Lasche (14) gegenüber dem Steg (4) geschwächt ist.

5. Sprunggelenkorthese nach einem der Ansprüche 1 bis 4, **dadurch gekennzeichnet, dass** die Schenkel (2, 3) mit dem Steg (4) und die Lasche (14) auf der dem Körper zugewandten Seite von einem Polsterstoff abgedeckt sind.

6. Sprunggelenkorthose nach einem der Ansprüche 1 bis 5, **dadurch gekennzeichnet, dass** der Steg (4) sich so weit in die Schenkel (2, 3) erstreckt, dass seine Länge an die Fußbreite des jeweiligen Patienten durch entsprechende Biegung des Stegs (4) gegenüber den Schenkeln (2, 3) anpassbar ist.

7. Sprunggelenkorthese nach einem der Ansprüche 1 bis 6, **dadurch gekennzeichnet, dass** an der Innenseite des Außenschenkels ein Polster (18) vorgesehen ist.

## Claims

1. Ankle orthosis comprising a U-shaped support stirrup (1) made of a flexible material, the sides (2, 3) of which stirrup converge beneath the foot in a web (4) projecting as a tab (14) toward the metatarsus, extend past the bones, and are held together at their end region by a fastening strap (6, 11) wrapping around the leg, **characterized in that** the tab (14) has a spherical convexity (15), which presses against the cuboid bone of the foot due to a preloading of the tab (14).

2. Ankle orthosis according to claim 1, **characterized in that** the tab (14) is formed as a projection that extends from the web (4) toward the forefoot along the outer sides of the sole of the foot, terminates short of the metatarsus, and is flexible relative to the web (4).

3. Ankle orthosis according to claim 1, **characterized in that** the tab (14) and the web (4) are made of different materials with different elasticity.

4. Ankle orthosis according to claim 1, **characterized in that** the tab (14) and the web (4) are made as a single part, wherein the tab (14) is weakened relative to the web (4).

5. Ankle orthosis according to any of the claims 1 to 4, **characterized in that** the sides (2, 3) with the web (4) and the tab (14) are covered by a padding material on the side facing the body.

6. Ankle orthosis according to any of the claims 1 to 5, **characterized in that** the web (4) extends far enough into the sides (2, 3) that its length can be matched to the foot width of the particular patient by appropriately bending the web (4) relative to the sides (2, 3).

7. Ankle orthosis according to any of the claims 1 to 6, **characterized in that** a pad (18) is provided on the inner surface of the outer side.

## Revendications

1. Orthèse pour l'articulation de la cheville qui contient un étrier-support (1) en forme de U composé d'un matériau souple, dont les montants (2, 3) convergent en un sous-pied (4) s'étendant en tant que languette (14) dans le sens du métatarse, dépassent la cheville et sont tenus ensemble dans leur extrémité par une bande de fixation (6, 11) enlaçant la jambe, **caractérisée en ce que** la languette (14) présente une voûte convexe (15), qui appuie sur l'os cuboïde en raison d'une tension préalable de la languette (14).

2. Orthèse pour l'articulation de la cheville selon revendication 1, **caractérisée en ce que** la languette (14) est placée en tant qu'avancée à partir du sous-pied (4) indiquant le sens de l'avant-pied, s'étendant le long des faces externes du pied de la plante de pied, qui termine avant le métatarse et peut être recourbée par rapport au sous-pied (4).

3. Orthèse pour l'articulation de la cheville selon revendication 1 ou 2, **caractérisée en ce que** la languette (14) et le sous-pied (4) sont constitués de différents matériaux avec une élasticité différente.

4. Orthèse pour l'articulation de la cheville selon revendication 1 ou 2, **caractérisée en ce que** la languette (14) et le sous-pied (4) sont formés en une pièce, sachant que la languette (14) est ramollie par rapport au sous-pied (4).

5. Orthèse pour l'articulation de la cheville selon l'une des revendications 1 à 4, **caractérisée en ce que** les montants (2, 3) avec le sous-pied (4) et la languette (14) sur le côté tourné vers le corps, sont recouverts d'un tissu rembourré.

6. Orthèse pour l'articulation de la cheville selon l'une des revendications 1 à 5, **caractérisé en ce que** le sous-pied (4) s'étend jusque dans les montants (2, 3) de manière à ce que sa longueur soit adaptable à la largeur de pied du patient concerné, par une courbure du sous-pied (4) par rapport aux montants (2, 3).

7. Orthèse pour l'articulation de la cheville selon l'une des revendications 1 à 6, **caractérisée en ce qu'**un rembourrage (18) est prévu sur la face interne du montant extérieur.
